# EUROPEAN PATENT SPECIFICATION

(11) **EP 4 208 229 B1**
(45) Date of publication and mention of the grant of the patent: **21.01.2026**
(21) Application number: 21772996.1
(22) Date of filing: 31.08.2021
(51) Int. Cl.: A61M 15/00

(54) **DRUG DELIVERY DEVICES WITH LOCK-OUT BEFORE DRUG DELIVERY**
ARZNEIMITTELABGABEVORRICHTUNGEN MIT SPERRUNG VOR DER ARZNEIMITTELABGABE
DISPOSITIFS D'ADMINISTRATION DE MÉDICAMENT AVEC VERROUILLAGE AVANT L'ADMINISTRATION DU MÉDICAMENT

(30) Priority: 01.09.2020 US 202063073019 P
(43) Date of publication of application: 12.07.2023
(60) Divisional application: 25221410.1
(73) Proprietor: JANSSEN Pharmaceutica NV, 2340 Beerse (BE)
(72) Inventor: SCRIMGEOUR, Ian, Dunbar EH42 ILT (GB); HUBERT, Emma, Louise, Milpitas, California 95035 (US); POPLI, Shagun, Milpitas, 95035 (US); CANNAMELA, Michael, New York 10001 (US); KAPIL, Monica, A., San Jose, California 95127 (US); VESOLE, Steven, M., Milpitas, California 95035 (US)
(74) Representative: Carpmaels & Ransford LLP
(86) International application number: PCT/EP2021/074018
(87) International publication number: WO 2022/049072

(56) References cited:
- WO-A1-2017/094027
- CA-A1- 2 256 008
- US-A- 5 944 222
- US-A1- 2010 258 118
- US-A1- 2018 263 283

## Description

### FIELD

The present disclosure relates generally to drug delivery devices with lock-out before drug delivery and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration. Additionally, drugs should typically only be delivered to a user from these devices at certain times or at certain time intervals in accordance with the user's prescription and/or instructions provided to the user for the drug's safe use. Deviating drug delivery from these certain times or time intervals may cause user harm by too-frequent drug delivery or too-infrequent drug delivery. Also, too-frequent drug delivery can be a particular problem for controlled substances.

Accordingly, there remains a need for improved nasal drug delivery devices.
US 5944222 A relates to a discharge apparatus for flowable media, comprising a basic body having a central, substantially cylindrical portion having upper and lower ends, and a protective sleeve to protect a connector portion against contamination.
CA 2256008 A1 relates to a dispenser for fluids, especially medicated fluids, from a mouthpiece aperture connected to a fluid supply by the manual operation of a piston-cylinder device, which releases the manual control only after an overload threshold has been exceeded.
US 2018/263283 A1 relates to a vape device system including a payload reservoir that is identified by a payload identifier and that is configured to hold a substance for atomization.
WO 2017/094027 A1 relates to a nasal spray assembly comprising a nasal device and a container, the container including a reservoir containing a medicament, the reservoir being sealed by a movable piston, the nasal spray assembly being for administering the medicament to the nose of a user, the nasal device comprising a body and a cap.
US 2010/258118 A1 relates to inhaler devices and a pharmaceutical dry powder composition to be dispensed using such inhaler devices for pulmonary administration.

### SUMMARY

In general, drug delivery devices with lock-out before drug delivery, drug products utilizing the same, and methods of using drug delivery devices with lock-out before drug delivery are provided. The invention is defined in the independent claims. Embodiments of the invention are defined in the dependent claims.

For example, the drug delivery device can include a depth guide, the dispensing head can include a distal base, and, with the locking mechanism and the drug delivery device releasably coupled together, the accessory is located between the depth guide and the distal base. For still another example, the accessory can include an opening therein that is configured to receive a tool therein that releases the coupling of the locking mechanism and the drug delivery device. For another example, the accessory can be a box that contains a partial portion of the drug delivery device therein when releasably coupled to the drug delivery device. For still another example, the accessory can be a box that fully contains the drug delivery device therein when releasably coupled to the drug delivery device.

For another example, the accessory can include a cavity formed therein, and, with the locking mechanism and the drug delivery device releasably coupled together, the drug delivery device can be seated in the cavity and can extend proximally from the accessory. The actuator can be configured to be seated in the cavity. The accessory can include an opening therein that is configured to receive a tool therein that releases the coupling of the locking mechanism and the drug delivery device. The accessory can include a measurement mechanism configured to facilitate a determination of an amount of drug contained in the drug holder. The measurement mechanism can include a spring configured to be compressed with the drug delivery device seated in the cavity, and an amount of the compression can be indicative of the amount of the drug contained in the drug holder. The drug holder can include a magnet thereon, and the measurement mechanism can include a Hall effect sensor.

For yet another example, the accessory can also include a time sensor, the locking mechanism can include a processor configured to automatically release the locking mechanism from the drug delivery device in response to time data gathered by the time sensor indicating that the time data is a predetermined acceptable time for delivery of the drug from the drug delivery device, the accessory can also include a memory storing predetermined acceptable times therein, and the memory can be accessible to the processor. For another example, the accessory can also include a location sensor, the locking mechanism can include a processor configured to allow the locking mechanism to be released from the drug delivery device in response to geographic location data gathered by the location sensor indicating that the geographic location data is a predetermined acceptable geographic location for delivery of the drug from the drug delivery device, the accessory can also include a memory storing predetermined acceptable geographic locations therein, and the memory can be accessible to the processor.

For yet another example, at least one of the drug delivery device and the accessory can include a communications interface configured to communicate with an external computer system, and the communications interface can be configured to receive an instruction from the external computer that allows the locking mechanism to be released from the drug delivery device. The instruction can be based on at least one of the drug delivery device and the accessory being in an acceptable geographic location, the instruction can be based on occurrence of a predetermined acceptable time for delivery of the drug from the drug delivery device, and/or the instruction can be based on an acceptable biometric input being input to at least one of the drug delivery device and the accessory.

For still another example, a code can be on the drug delivery device, the accessory can also include a reader configured to read the code, the locking mechanism can include a processor configured to allow the locking mechanism to be released from the drug delivery device in response to the read code indicating that the code is a predetermined acceptable code for the drug delivery device, the accessory can also include a memory storing predetermined acceptable codes therein, and the memory can be accessible to the processor. For yet another example, the releasable coupling can be mechanical. For still another example, the releasable coupling can be electrical. For another example, the drug is one of ketamine, esketamine, naloxone, and sumatriptan.

**In** another aspect, a drug product is provided that in one embodiment includes a drug product disposed in a drug delivery device. The drug delivery device includes a dispensing head including a tip configured to be positioned in a nose of a patient. The tip has an opening therein. The drug delivery device also includes a drug holder containing the drug product therein, and an actuator configured to be actuated by a user to cause the drug product to be delivered out of the opening. The drug product is one of ketamine, esketamine, naloxone, and sumatriptan. The drug delivery device can have any number of variations.

In another embodiment, a drug delivery system includes a drug delivery device and an accessory. The drug delivery device includes a dispensing head including a tip configured to be positioned in a nose of a patient. The tip has an opening therein. The drug delivery device also includes a drug holder containing a drug therein, and an actuator configured to be actuated to cause the drug to be delivered out of the opening. The accessory is a separate, independent member from the drug delivery device that is configured to releasably couple to the drug delivery device. The accessory and the drug delivery device can only be decoupled from one another by manual user action, and the manual user action includes one of insertion of an unlocking tool into a tool opening formed in the accessory, unlocking of a numerical and/or alphabetical combination lock, and providing a biometric input.

The drug delivery system can have any number of variations. For example, the manual user action can include insertion of the unlocking tool into the tool opening formed in the accessory, and the insertion of the unlocking tool can release a spring-engaged mechanism to allow for the decoupling of the accessory and the drug delivery device. For another example, the manual user action can include unlocking of the numerical and/or alphabetical combination lock. For yet another example, the manual user action can include providing the biometric input, and the accessory can also include a processor configured to determine whether the biometric input is an acceptable biometric input and, if so, to allow decoupling of the accessory and the drug delivery device, and if not, to not allow the decoupling of the accessory and the drug delivery device. For still another example, with the accessory and the drug delivery device releasably coupled together, the actuator can be prevented from being actuated, and, with the accessory and the drug delivery device not releasably coupled together, the actuator can be allowed to be actuated.

For another example, the drug delivery device can also include a viewing window configured to allow a user to visualize a status of drug delivery from the drug delivery device, with the accessory and the drug delivery device releasably coupled together the viewing window can be obscured such that the user cannot use the viewing window to visualize the status of drug delivery from the drug delivery device, and with the accessory and the drug delivery device decoupled from one another the viewing window can not be obscured such that the user can use the viewing window to visualize the status of drug delivery from the drug delivery device. The accessory can also include a protrusion, with the accessory and the drug delivery device releasably coupled together the protrusion can be seated in the viewing window, and with the accessory and the drug delivery device decoupled from one another the protrusion can not be seated in the viewing window.

For yet another example, the accessory can include a cavity formed therein, and, with the accessory and the drug delivery device releasably coupled together, the drug delivery device can be seated in the cavity and can extend proximally from the accessory. For another example, the accessory can be a box that contains a partial portion of the drug delivery device therein when releasably coupled to the drug delivery device. For still another example, the accessory can be a box that fully contains the drug delivery device therein when releasably coupled to the drug delivery device. For another example, the drug can be one of ketamine, esketamine, naloxone, and sumatriptan.

In another aspect, a drug product is provided that in one embodiment includes a drug product disposed in a drug delivery device. The drug delivery device includes a dispensing head including a tip configured to be positioned in a nose of a patient. The tip has an opening therein. The drug delivery device also includes a drug holder containing the drug product therein, and an actuator configured to be actuated to cause the drug product to be delivered out of the opening. The drug product is one of ketamine, esketamine, naloxone, and sumatriptan. The drug delivery device can have any number of variations.

In another aspect, a drug delivery method is provided that in one embodiment includes releasing, via a manual user action, a nasal drug delivery device from an accessory that is a separate, independent member from the drug delivery device. The drug delivery method also includes, after the release, actuating an actuator of the drug delivery device and thereby causing a drug contained in a drug holder of the drug delivery device to exit through an opening and into a nose.

### BRIEF DESCRIPTION OF DRAWINGS

The present invention is described by way of reference to the accompanying figures which are as follows:
FIG. 1 is a block diagram of one embodiment of a drug delivery device;
FIG. 2 is a block diagram of one embodiment of a drug delivery device and an accessory configured to releasably couple together;
FIG. 3 is a perspective view of one embodiment of the drug delivery device and the accessory of FIG. 2;
FIG. 4 is a side cross-sectional view of another embodiment of a drug delivery device;
FIG. 5 is a perspective view of another embodiment of the drug delivery device and the accessory of FIG. 2;
FIG. 6 is a perspective view of the accessory of FIG. 5;
FIG. 7 is a perspective view of yet another embodiment of the drug delivery device and the accessory of FIG. 2;
FIG. 8 is a perspective view of still another embodiment of the drug delivery device and the accessory of FIG. 2;
FIG. 9 is a perspective view of another embodiment of the drug delivery device and the accessory of FIG. 2;
FIG. 10 is a perspective view of one embodiment of the drug delivery device and the accessory of FIG. 9;
FIG. 11 is a perspective view of still another embodiment of the drug delivery device and the accessory of FIG. 2;
FIG. 12 is a perspective view of another embodiment of the drug delivery device and the accessory of FIG. 2; and
FIG. 13 is a schematic view of one embodiment of a computer system.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with lock-out before drug delivery, drug products utilizing the same, and methods of using drug delivery devices with lock-out before drug delivery are provided. **In** general, a nasal drug delivery device is configured to dispense a drug therefrom into a nose and is configured to be releasably coupled to an accessory. The accessory is a separate, independent member from the drug delivery device such that in its decoupled state, the accessory is completely removed from the drug delivery device. With the drug delivery device and the accessory releasably coupled together, one or more safety features may be achieved. For example, the drug delivery device and the accessory being releasably coupled together may prevent the drug from being accessed by an unauthorized party, such as a party who has not been prescribed the drug. Preventing such access may be particularly important for controlled substances. For another example, the drug delivery device and the accessory being releasably coupled together may prevent the drug from being accessed too frequently, from being accessed out of accordance from a prescription for the drug, and/or from being accessed contrary to instructions provided to a user for the drug's safe use. Preventing such access can help keep a user's drug deliveries on schedule and/or can be particularly important for controlled substances. For yet another example, the drug delivery device and the accessory being releasably coupled together may provide child resistance such that a child cannot access the drug, similar to the child resistance provided by pill bottles with child resistant caps. For still another example, the drug delivery device and the accessory being releasably coupled together may allow a user's health care provider to be informed when the drug delivery device is in use by being electronically informed that the drug delivery device and the accessory have been decoupled from one another. The health care provider may therefore be better able to analyze user treatment and/or user compliance and thereby improve user care.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient can have a variety of configurations. In general, the drug delivery device is configured to deliver a drug to a patient, where the drug is provided in a defined dosage form within the drug delivery device.

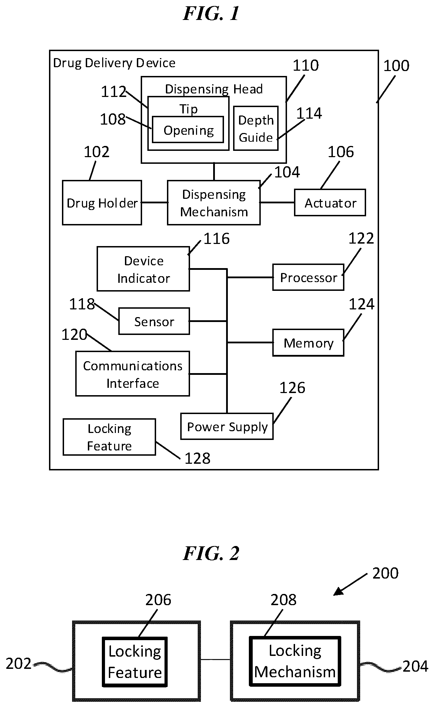

FIG. 1 illustrates one embodiment of a drug delivery device 100 configured to expel a drug into a nose of a patient and to releasably couple to an accessory using a locking feature 128. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, such as a cartridge, a vial, a blow-fill-seal (BFS) capsule, a blister pack, etc. In an exemplary embodiment, the drug holder 102 is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston and/or a needle configured to pierce through or puncture a seal member of the drug holder 102 in embodiment in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a pressable button. For another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100 in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

In some embodiments, the dispensing head 110 can include two tips 112 each having an opening 108 therein such that the drug delivery device 100 is configured to simultaneously deliver doses of drug into two nostrils in response to a single actuation of the actuator 106.

In some embodiments, the actuator 106 is configured to be actuated only once to deliver a dose of the drug to a patient.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

**In** embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or and to be communicated externally via the communications interface 120. The memory 124 is configured to store instructions that are configured to be executed by the processor 122 for the processor 122 to process information regarding the various electrical components with which the processor 122 is in communication.

The locking feature 128 is configured to releasably couple the drug delivery device 100 to an accessory that is a separate, independent member from the drug delivery device 100. In general, the locking feature 128 is configured to have a locked configuration and an unlocked configuration. In the locked configuration, the locking feature 128 locks the drug delivery device 100 to the accessory such that the drug delivery device 100 and the accessory are a mated unit. The drug delivery device 100 can be prevented from being actuated to deliver drug therefrom with the locking feature 128 in the locked configuration. In the unlocked configuration, the locking feature 128 does not lock the drug delivery device 100 to the accessory such that the drug delivery device 100 is a standalone unit with respect to the accessory and can be used by a user to deliver drug from the drug delivery device 100. As discussed further below, the locking feature 128 is configured to move from the locked configuration to the unlocked configuration to allow for use of the drug delivery device 100.

The locking feature 128 can have a variety of configurations. In an exemplary embodiment, the locking feature 128 is a mechanical feature configured to mechanically engage the accessory to mate and lock the drug delivery device 100 to the accessory in the locked configuration of the locking feature 128 and to mechanically disengage from the accessory to unlock the drug delivery device 100 from the accessory in the unlocked configuration of the locking feature 128. In another exemplary embodiment, the locking feature 128 is an electrical feature configured to electrically engage the accessory to mate and lock the drug delivery device 100 to the accessory in the locked configuration of the locking feature 128 and to electrically disengage from the accessory to unlock the drug delivery device 100 from the accessory in the unlocked configuration of the locking feature 128.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

FIG. 2 illustrates an exemplary embodiment of a system 200 including a drug delivery device 202, e.g., the drug delivery device 100 of FIG. 1, and an accessory 204 configured to releasably lock with the drug delivery device 202. The drug delivery device 202 includes a locking feature 206, e.g., the locking feature 128 of FIG. 1, and the accessory 204 includes a locking mechanism 208 configured to releasably mate with the locking feature 206. In a locked configuration, the locking feature 206 and the locking mechanism 208 are mated to one another such that the drug delivery device 202 and the accessory 204 are a mated unit and the drug delivery device 202 is prevented from being actuated to deliver drug therefrom. In an unlocked configuration, the locking feature 206 and the locking mechanism 208 are not mated to one another such that the drug delivery device 202 and the accessory 204 are not a mated unit and the drug delivery device 202 is allowed to be actuated to deliver drug therefrom.

In an exemplary embodiment, the drug delivery device 202 is configured to be disposable, and the accessory 204 is configured to be reusable. The accessory 204 can thus be configured to be reused with multiple drug delivery devices 202. After the drug delivery device 202 is unlocked from the accessory 204, a second drug delivery device 202 can be locked to the accessory 204 and so on. An authorized user of the drug delivery device 202, e.g., a patient with a prescription for the drug delivered therefrom, will often use multiple drug delivery devices 202 over the course of the user's treatment as the user receives doses of the drug over time from different drug delivery devices. The accessory 204 being reusable allows the user to use the same accessory 204 with each of the user's drug delivery devices 202, which may help familiarize the user with the accessory 204 and/or the user's treatment regimen, and/or may reduce user, insurer, hospital, etc. costs since only one accessory 204 per user need be provided.

Disposal of the drug delivery device 202 can be final discarding of the drug delivery device 202, e.g., as medical waste, or can be recycling of the drug delivery device 202. Some drugs, such as esketamine, ketamine, and other controlled substances, may be required to be disposed of per a government requirement, e.g., U.S. federal Drug Enforcement Administration requirement, non-U.S. federal requirement, state requirement, or local requirement, to help, e.g., ensure that the drug is not accessed by an unauthorized party. Even if the drug delivery device 202 is used properly and delivers such a drug therefrom, disposal of the drug delivery device 202 may still be required, e.g., because a residual amount of the drug may be left in the drug delivery device 202 that could potentially be accessed by an unauthorized party or by the user after proper use of the drug delivery device 202.

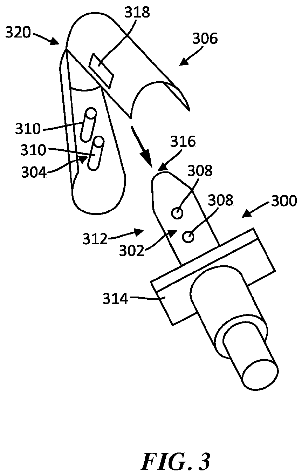

FIG. 3 illustrates an exemplary embodiment of the drug delivery device 202 and the accessory 204 of FIG. 2. FIG. 3 illustrates a drug delivery device 300 that includes a locking feature 302 configured to releasably mate to a locking mechanism 304 of an accessory 306. In this illustrated embodiment, the locking feature 302 and the locking mechanism 304 are configured to mechanically engage to lock the drug delivery device 300 and the accessory 306 together and to mechanically disengage to unlock the drug delivery device 300 from the accessory 306.

The locking feature 302 includes a plurality of depressions 308, and the locking mechanism 304 includes a plurality of protrusions 310 configured to seat in the depressions 308. There are a same number of depressions 308 and protrusions 310 such that one protrusion 310 seats in each of the depressions 308. In other embodiments, there can be only one depression and only one protrusion, and/or the locking feature 302 can include one or more protrusions and the locking mechanism 304 can include one or more depressions. In this illustrated embodiment, the depressions 308 have a circular cross-sectional shape and the protrusions 310 are cylindrical pins, but the depressions 308 and the protrusions 310 can have other shapes complementary to one another, e.g., the depressions 308 being linear grooves and the protrusions 310 being rectangular pins, the depressions 308 having an ovular cross-sectional shape and the protrusions 310 being pins with an ovular cross-sectional shape, etc.

The locking feature 302 is located on a dispensing head 312 of the drug delivery device 300 proximal to a distal base 314 of the dispensing head 312 and distal to the drug delivery device's opening 316. The accessory 306 is configured to be releasably mated to the drug delivery device 300, e.g., with the locking feature 302 and the locking mechanism 304 mated together, such that the accessory 306 covers the opening 316. Drug in a drug holder of the drug delivery device 300 therefore cannot exit the opening 316 and enter a patient's nose even if the drug delivery device 300 is actuated. The accessory 306 can be fluid tight in the locked configuration to prevent access to any drug that exits the opening 316 with the accessory 306 and the drug delivery device 300 locked together.

The accessory 306 includes a tool opening 318 configured to receive an unlocking tool therein. The unlocking tool includes a distal tip or other portion thereof that is configured to be inserted into the tool opening 318 and release a spring-engaged mechanism of the accessory 306 by pushing thereon. The release of the spring-engaged mechanism causes the accessory 306 to move from a closed state, in which the accessory 306 is locked to the drug delivery device 300, to an open state, in which the accessory 306 is unlocked from the drug delivery device 300. FIG. 3 illustrates the accessory 306 in the open state. The accessory 306 is biased to the open state by the spring-engaged mechanism. The release of the spring-engaged mechanism thus automatically causes the accessory 306 to move from the closed state to the open state. The accessory 306 includes a hinge 320 at which the accessory 306 is configured to pivot open and closed. In other embodiments, instead of a spring-engaged mechanism, the unlocking tool can be configured to be inserted into the tool opening 318 and release a mechanical clip of the accessory 306 by pushing thereon. In other embodiments, the unlocking tool can include a magnetic element, e.g., one or more magnets embedded therein and/or on an exterior surface thereof, that is configured to release the mechanical clip, which in such embodiments is formed of metal attracted to the magnetic element in at least a portion of the clip configured to open to release the clip.

The tool opening 318 and the insertable portion of the unlocking tool have complementary shapes and sizes, similar to a lock and key. The complementary shape is rectangular in this illustrated embodiment, but other shapes can be used such as circular, X-shaped, triangular, zig-zag shaped, C-shaped, etc.

The drug delivery device 300 in the illustrated embodiment of FIG. 3 is not powered, e.g., does not include any electrical components such as a processor, a sensor, a memory, a communications interface, etc. However, a powered drug delivery device can be configured to releasably mate to the accessory 306 similar to the drug delivery device 300.

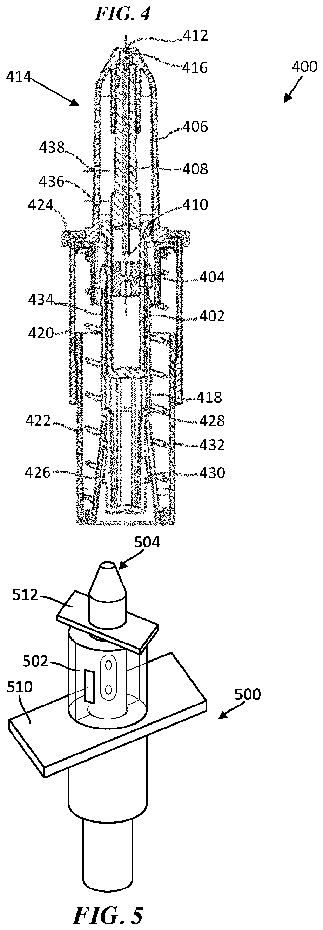

FIG. 4 illustrates a drug delivery device 400 that is an exemplary embodiment of the drug delivery device 300 of FIG. 3. The drug delivery device 400 is generally configured and used similar to that discussed above regrading FIGS. 1 and 3 and includes a drug holder 402 containing two doses of drug therein. A dispenser member, such as a piston 404, is mounted to slide in the drug holder 402. In a pre-actuation position of the drug delivery device 400, shown in FIG. 4, the piston 404 acts as a stopper, isolating the drug. The drug delivery device 400 also includes a dispensing head 406 that is assembled on the drug holder 402 and that is configured to be axially movable relative to the drug holder 402. In particular, an axial movement of the dispensing head 406 relative to the drug holder 402 causes the piston 404 to move in the drug holder 402, and thus causes the drug contained in the drug holder 402 to be dispensed. The dispensing head 406 includes a dispenser channel 408 that extends from a perforator tip 410 to an opening 412 in a tip 414 of the dispensing head 406. Proximal to or upstream of the opening 412, a spray profile 416 can be provided that is configured to dispense the drug in the form of spray.

The drug holder 402 is fastened in a body 418 that is thus secured to the drug holder 402 and that moves together with the drug holder 402.

The dispensing head 406 includes a bottom side skirt 420 that is adapted to cooperate with an actuator 422 of the drug delivery device 400. A finger-rest element 424 can be assembled around the dispensing head 406 or can be formed integrally therewith.

The actuator 422 is configured to be axially movable inside the side skirt 420 of the dispensing head 406 so as to perform successive actuations of the drug delivery device 400. The actuator 422 includes at least one sloping tab 426 that is configured to cooperate with projections 428, 430 of the body 418 so as to perform successive actuations. A return spring 432 is mounted between the actuator 422 and the dispensing head 406 so as to return the actuator 422 into its start position after each actuation.

The drug delivery device 400 includes a device indicator 434 configured to indicate to a user whether a first dose has been dispensed and whether a second dose has been dispensed. In this way, the user knows exactly what the situation is, and whether or not the first dose has been dispensed. The indicator 434 is configured to cooperate with viewing windows 436, 438 that are formed in the dispensing head 406. The viewing windows 436, 438 are formed in a side wall of the dispensing head 406, clearly visible to the user when the drug delivery device 400 is held in the hand.

The viewing windows 436, 438 can serve as the drug delivery device's locking feature, e.g., as the locking feature 302, with a locking mechanism of an accessory being releasably mateable therewith. The viewing windows 436, 438 will thus be obscured and not visible to a user in embodiments in which the accessory is opaque at least in an area that covers the viewing windows 436, 438 when the drug delivery device 400 and the accessory are locked together.

The drug delivery device 400 is further described in U.S. Pat. No. 9,555,950 entitled "Fluid Product Dispensing Device" issued Jan. 31, 2017.

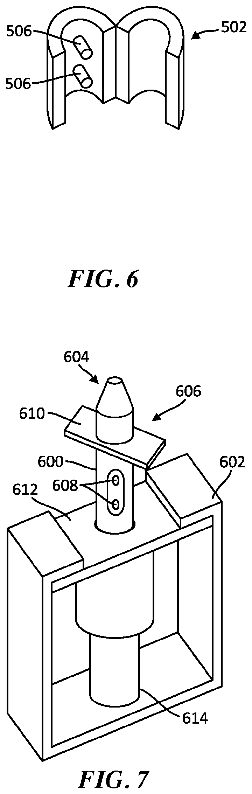

FIG. 5 illustrates another exemplary embodiment of the drug delivery device 202 and the accessory 204 of FIG. 2. In this illustrated embodiment, a drug delivery device 500 and an accessory 502 are generally configured and used similar to the drug delivery device 300 and the accessory 306 of FIG. 3. FIG. 5 illustrates the drug delivery device 500 and the accessory 502 locked together. FIG. 6 illustrates the accessory 502 as a standalone element in its open state with its locking mechanism in the form of a pair of protrusions 506 extending radially inward from an interior surface thereof. However, in this illustrated embodiment, the accessory 502 does not cover an opening 504 formed in a dispensing head 508 of the drug delivery device 500. Instead, the accessory 502 is configured to be releasably mated to the drug delivery device 500 proximal to a distal base 510 of the dispensing head 506 and distal to the drug delivery device's depth guide 512 that is located distal to the opening 504. The drug delivery device 400 of FIG. 4 is also an exemplary embodiment of the drug delivery device 500 of FIG. 5.

FIG. 7 illustrates another exemplary embodiment of the drug delivery device 202 and the accessory 204 of FIG. 2. In this illustrated embodiment, a drug delivery device 600 and an accessory 602 are generally configured and used similar to the drug delivery device 300 and the accessory 306 of FIG. 3. FIG. 7 illustrates the drug delivery device 600 and the accessory 602 locked together. However, in this illustrated embodiment, the accessory 602 does not cover an opening 604 formed in a dispensing head 606 of the drug delivery device 600, and the drug delivery device's locking feature does not include a depression and does not include the drug delivery device's pair of viewing windows 608. Instead, the accessory 602 is configured to be releasably mated to the drug delivery device 600 distal to a depth guide 610 of the dispensing head 606, proximal to a distal base 612 of the dispensing head 606, and distal to the drug delivery device's actuator 614. The accessory 602, when locked to the drug delivery device 600, is configured to prevent actuation of the drug delivery device 600 by preventing user access to the distal base 612 of the dispensing head 606 and the actuator 614, which are configured to be held by a user during actuation.

The accessory 602 is configured to lock around the drug delivery device 600 similar to the accessories 306, 502 of FIGS. 3, 5, and 6. However, the accessory 602 in this illustrated embodiment is configured as a rectangular box that surrounds a partial portion of the drug delivery device 600. The box can have other shapes, e.g., pyramidal, a half dome, a cube, etc.

The accessory 602 can, in other embodiments, surround the entirety of the drug delivery device 600. In such embodiments, a single drug delivery device 600 can be contained in the accessory 602 such that the accessory 602 unlocking allows user access to the one drug delivery device 600. The accessory 602 can, however, have a plurality of drug delivery devices 600 contained therein. In such embodiments, the accessory 602 unlocking can allow access to all of the drug delivery devices 600 contained therein, or the accessory 602 unlocking can allow access to only one of the drug delivery devices 600 at a time, e.g., by having different compartments that are configured to be unlocked one at a time. Regardless of whether the accessory 602 contains one or more drug delivery devices 600 therein, the accessory 602 can be configured to receive each used drug delivery device 600 back therein, which may facilitate disposal of the drug delivery device(s) 600 by providing a convenient, safe transport package for the drug delivery device(s) 600.

The accessory 602 can be released from the drug delivery device 600 in any of a variety of ways. For example, the accessory 602 can include a tool opening similar to the tool opening 318 of FIG. 3. For another example, the accessory 602 can include a numerical and/or alphabetical combination lock thereon. For yet another example, the accessory 602 can be configured to unlock in response to a fingerprint, facial image (for face recognition), or other biometric input thereto. In such embodiments, the accessory 602 includes sufficient electronic components to implement the biometric lock, as will be appreciated by a person skilled in the art.

The drug delivery device 400 of FIG. 4 is also an exemplary embodiment of the drug delivery device 600 of FIG. 7.

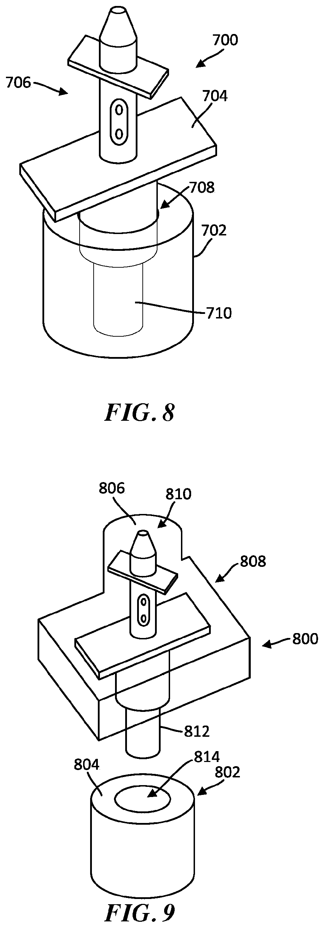

FIG. 8 illustrates another exemplary embodiment of the drug delivery device 202 and the accessory 204 of FIG. 2. In this illustrated embodiment, a drug delivery device 700 and an accessory 702 are generally configured and used similar to the drug delivery device 600 and the accessory 602 of FIG. 7. FIG. 8 illustrates the drug delivery device 700 and the accessory 702 locked together. However, in this illustrated embodiment, the accessory 702 is configured to be releasably mated to the drug delivery device 700 distal to a distal base 704 of the drug delivery device's dispensing head 706. Also, the accessory 702 in this illustrated embodiment is configured as a cylindrical member with a cavity 708 formed in a proximal end thereof that is configured to seat the drug delivery device's actuator 710 therein. The accessory 702, when locked to the drug delivery device 700, is configured to prevent actuation of the drug delivery device 700 by preventing user access to the actuator 710.

The accessory 702 can be released from the drug delivery device 700 in any of a variety of ways. For example, the accessory 702 can be configured to be released from the drug delivery device 700 using a child safety lock similar to child safety locks used with pill caps and bill bottles. For another example, the accessory 702 can include a tool opening similar to the tool opening 318 of FIG. 3. For still another example, the accessory 702 can include a numerical and/or alphabetical combination lock thereon. For yet another example, the accessory 702 can be configured to unlock in response to a fingerprint or other biometric input thereto. In such embodiments, the accessory 702 includes sufficient electronic components to implement the biometric lock, as will be appreciated by a person skilled in the art.

The drug delivery device 400 of FIG. 4 is also an exemplary embodiment of the drug delivery device 700 of FIG. 8.

FIG. 9 illustrates another exemplary embodiment of the drug delivery device 202 and the accessory 204 of FIG. 2. In this illustrated embodiment, a drug delivery device 800 and an accessory 802 are generally configured and used similar to the drug delivery device 700 and the accessory 702 of FIG. 8. However, in this illustrated embodiment, the accessory 802 includes, in addition to a distal member 804 configured and used similar to the accessory 702 of FIG. 8, a proximal member 806 configured to be releasably mated to the drug delivery device 800 on a dispensing head 808 of the drug delivery device 800 such that the proximal locking member 806 covers the drug delivery device's opening 810. The proximal member 806 is configured and used similar to the accessory 306 of FIG. 3. FIG. 9 illustrates the drug delivery device 800 and the distal member 804 unlocked from one another and the drug delivery device 800 and the proximal member 806 locked together.

In some embodiments, the distal and proximal members 804, 806 are configured to be independently released from the drug delivery device 800, as discussed above. The accessory 802 is thus configured to provide two separate locks of the drug delivery device 800, one provided by the distal member 804 and one provided by the proximal member 806. If one or both of the distal member 804 and the proximal member 806 is coupled to the drug delivery device 800, drug delivery is prevented. In other embodiments, the seating of the distal member 804 is configured to cause the unlocking of the proximal member 806. In such embodiments, the accessory 802 provides one lock of the drug delivery device 800 via the proximal member 806, with the distal member 804 acting as a key to unlock the proximal member 806 from the drug delivery device 800. For example, the distal member 804 can include a magnet, e.g., one magnet, two magnets, three magnets, etc., configured to act on a spring-engaged mechanism of the proximal member 806. In response to the drug delivery device 800, e.g., the drug delivery device's actuator 812, being mated to the distal member 804, e.g., seated in the distal member's cavity 814, the magnet's magnetic field can cause the spring-engaged mechanism to open and thereby move the proximal member 806 from a closed state, which is shown in FIG. 9, to an open state.

The drug delivery device 400 of FIG. 4 is also an exemplary embodiment of the drug delivery device 800 of FIG. 9.

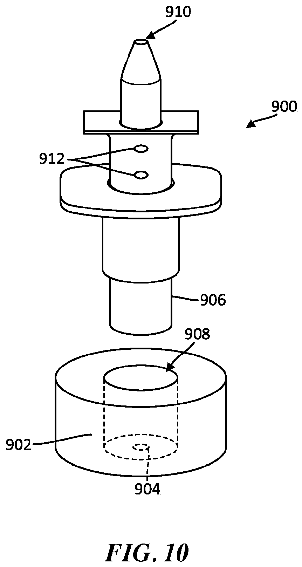

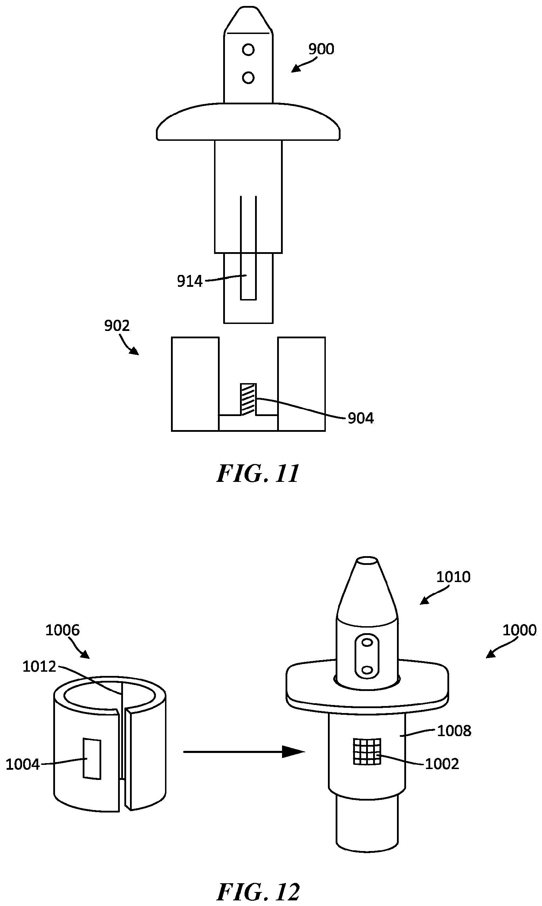

FIG. 10 illustrates another exemplary embodiment of the drug delivery device 202 and the accessory 204 of FIG. 2. In this illustrated embodiment, a drug delivery device 900 is generally configured and used similar to the drug delivery devices 700, 800 of FIGS. 8 and 9, and an accessory 902 is generally configured and used similar to the accessory 702 of FIG. 8 and the distal member 804 of FIG. 9. However, in this illustrated embodiment, the accessory 902 is configured to releasably couple to the drug delivery device 900 and includes a measurement mechanism 904 configured to facilitate a determination of an amount of drug contained in the drug delivery device's drug holder 914 (see FIG. 11) that contains the drug therein. In other words, the accessory 902 is configured to help determine the drug delivery device's fill level status. FIG. 11 also illustrates the drug delivery device 900 and an embodiment of the accessory 902 discussed further below.

In an exemplary embodiment, the measurement mechanism 904 of the accessory 902 is configured to use the Hall effect, in which a potential difference is produced across an electrical conductor when a magnetic field is applied in a direction perpendicular to that of the flow of current. For example, the measurement mechanism can include a Hall effect sensor configured to operatively connect to a magnet located on a bottom of the drug delivery device's drug holder 914. When the drug delivery device 900 is mated to the accessory 902, e.g., by an actuator 906 of the drug delivery device 900 being seated in a cavity 908 of the accessory 902, the electronics are configured to determine a distance thereof from the magnet. The distance defines how much of the drug is contained in the drug holder 914 and thus in the drug delivery device 900 since the drug holder 914 is configured to move proximally within the drug delivery device 900 with each actuation of the actuator 906. The electronics can include a memory storing data therein, such as in a lookup table, correlating distances with drug amounts, that a processor of the electronics is configured to use to determine the drug amount remaining in the drug holder 914. Before the actuator 906 is actuated for a first time, the amount of drug in the drug holder 914 is at a maximum amount, e.g., an initial fill amount. With each actuation of the actuator 906, the amount of drug released through the drug delivery device's opening 910 is a known, predetermined amount with the drug holder 914 moving a known, predetermined proximal travel distance with each actuation.

In another exemplary embodiment, shown in FIG. 11, the measurement mechanism 904 of the accessory 902 includes electronics and a spring. The spring is configured to contact a distal surface of the drug delivery device 900, e.g., a distal surface of the actuator 906 seated in the cavity 908. The spring is biased proximally. A compression of the spring thus indicates how much the drug holder has moved proximally and thus how much drug remains in the drug holder. The electronics are operatively coupled to the spring and are configured to determine the amount of spring compression, e.g., using a processor, a memory, and a sensor such as a pressure sensor, a Hall effect sensor, etc.

Regardless of the configuration of the measurement mechanism 904, the determined amount of drug in the drug holder may be useful to confirm a number of drug doses delivered by the drug delivery device 900. As discussed above, the drug delivery device's viewing windows 912 can be visually observed to indicate how many times the drug delivery device 900 has been actuated, either zero times, one time, or two times. The determined amount of the drug in the drug holder can electronically verify the number of actuations. The electronics of the accessory 902 include a device indicator configured to indicate the determined amount of drug, such as by visually indicating via a display screen, one or more lights, etc. a number of drug doses corresponding to the determined drug amount. Alternatively or in addition, the electronics of the accessory 902 can include a communications interface configured to communicate with a communications interface of an external computer system that is external to and separate from the accessory 902 and the drug delivery device 900. The external computer system can include a mobile phone, an electronic tablet, etc. The external computer system can be configured to indicate the determined amount of drug, such as by visually indicating via a display screen, one or more lights, etc. a number of drug doses corresponding to the determined drug amount. A user of the external computer system can be the user associated with the drug delivery device, and/or a user of the external computer system can be a party other than the user associated with the drug delivery device, such as the patient's health care provider. In instances in which the user of the external computer system is a party other than the user of the drug delivery device, the determined drug amount indicated by the external computer system can allow the party to be remotely located from the user of the drug delivery device who cannot visually see the viewing windows 912 to verify drug delivery. Some drugs, such as esketamine, ketamine, and other controlled substances, may require verification of drug delivery by a health care provider per the drug's Risk Evaluation and Management Strategies (REMS), so remote verification of drug delivery may allow for more flexibility in locations for use of the drug delivery device 900.

In some embodiments, the accessory 902 of FIG. 10 does not include a locking function like the accessory 702 of FIG. 8 and the distal member 804 of FIG. 9 but does include the above-discussed drug amount determination function.

The drug delivery device 400 of FIG. 4 is also an exemplary embodiment of the drug delivery device 900 of FIG. 10.

FIG. 12 illustrates another exemplary embodiment of the drug delivery device 202 and the accessory 204 of FIG. 2. FIG. 12 illustrates a drug delivery device 1000 that includes a locking feature 1002 configured to releasably mate to a locking mechanism 1004 of an accessory 1006. FIG. 12 illustrates the drug delivery device 1000 and the accessory 1006 unlocked from one another. The drug delivery device 1000 and the accessory 1006 in this illustrated embodiments are generally configured and used similar to the drug delivery device 500 and the accessory 502 of FIGS. 5 and 6. However, in this illustrated embodiment, the locking feature 1002 and the locking mechanism 1004 are configured to electrically engage to lock the drug delivery device 1000 and the accessory 1006 together and to electrically disengage to unlock the drug delivery device 1000 from the accessory 1006.

The locking feature 1002 and the locking mechanism 1004 can have a variety of configurations. In an exemplary embodiment, the locking feature 1002 includes a code, e.g., a bar code, a QR code, RFID tag data, serial number, Drug Enforcement Administration (DEA) Registration Number, etc., printed on, etched in, etc. on the drug delivery device 1000 or otherwise attached to the drug delivery device 1000 such as on a label or sticker on the drug delivery device 1000. The locking feature 1002 is on a body 1008 of the drug delivery device 1000 distal to the drug delivery device's dispensing head 1010 in this illustrated embodiment but can be in another location. The locking mechanism 1004 includes electronics configured to read the code when the accessory 1006 is locked to the drug delivery device 1000. The electronics include a reader, e.g., a bar code reader, an RFID reader, a QR code scanner, a camera, etc., configured to capture data from the locking feature 1002 that is indicative of the code. The electronics also include a processor configured to determine from the data gathered by the reader that the drug delivery device 1000 is a verified drug delivery device. The processor can determine that the drug delivery device 1000 is a verified drug delivery device in a variety of ways, such as by comparing the gathered data with data stored in a memory of the electronics that identifies verified drug delivery device codes. If the drug delivery device 1000 is a verified drug delivery device, the accessory 1006 can automatically allow the accessory 1006 to be released from drug delivery device 1000, e.g., by the processor causing the accessory 1002 to move from its closed state to its open state by moving a switch operatively engaged with the accessory's hinge 1012, which can be spring-loaded to bias the accessory 1006 to the open state. If the drug delivery device 1000 is a not verified drug delivery device, the accessory 1006 can prevent the accessory 1006 from being released from drug delivery device 1000.

In addition to or instead of the accessory 1006 being configured to use drug delivery device 1000 identification data in allowing or preventing accessory 1006 release from the drug delivery device 1000, the accessory 1006 can be configured to use at least one other type of data in allowing or preventing accessory 1006 release from the drug delivery device 1000. For example, the electronics of the accessory 1006 can include a location sensor configured to monitor geographic location, e.g., via satellite position determination, such as GPS. The processor of the electronics can be configured to determine, based on the location data sensed by the location sensor and communicated to the processor, whether the sensed geographic location is an acceptable geographic location or an unacceptable geographic location. An acceptable geographic location is a location within the drug delivery device's expected supply chain and/or a predetermined location of a patient prescribed the drug in the drug delivery device 1000. The acceptable geographic locations for particular drug delivery devices will vary, as different drug delivery devices are used by different people and are shipped using different shipping channels based on factors such as a drug delivery device's intended end location, the manufacturer of the drug delivery device, etc. The acceptable geographic locations for a particular drug delivery device can be programmed into a memory of the electronics. The processor is configured to use the stored acceptable geographic locations to determine whether or not the sensed geographic location is acceptable, e.g., by using a lookup table. If the geographic location is an acceptable geographic location, the accessory 1006 can automatically allow the accessory 1006 to be released from drug delivery device 1000. If the geographic location is an unacceptable geographic location, the processor is configured to prevent the accessory 1006 from being released from drug delivery device 1000.

For another example, the electronics of the accessory 1006 can include a time sensor (e.g., a timer or clock device such as an atomic clock) configured to monitor time. The processor of the electronics can be configured to determine, based on the time data sensed by the time sensor and communicated to the processor, whether the sensed time is an acceptable time or an unacceptable time. An acceptable time is a day and time, which can be a discrete time or a range of times, within the drug delivery device's expected use of the drug delivery device 1000 according to, e.g., a patient's drug prescription, a last time that the drug delivery device 1000 was actuated to deliver drug therefrom, etc. The acceptable times for particular drug delivery devices will vary, as different drug delivery devices are used to deliver different drugs and are used by different people with different drug treatment regimens. The acceptable times for a particular drug delivery device can be programmed into a memory of the electronics. The processor is configured to use the stored acceptable times to determine whether or not the sensed time is acceptable, e.g., by using a lookup table. If the time is an acceptable time, the accessory 1006 can automatically allow the accessory 1006 to be released from drug delivery device 1000. If the time is an unacceptable time, the processor is configured to prevent the accessory 1006 from being released from drug delivery device 1000.

For yet another example, the electronics of the accessory 1006 can be configured to cause the accessory 1006 to unlock from the drug delivery device 1000 in response to a fingerprint, facial image (for face recognition), or other biometric input thereto. In such embodiments, the accessory 1006 includes sufficient electronic components to implement the biometric lock, as will be appreciated by a person skilled in the art.

For still another example, the electronics can include a communications interface configured to communicate with an external computer system and receive from the external computer system data indicating that a training device, e.g., a trainer for the drug delivery device 1000, has been successfully used. Successful use of the training device indicates that the user is ready to use the drug delivery device 1000 to deliver drug therefrom.

For another example, the electronics of the accessory 1006 can be configured to cause the accessory 1006 to unlock from the drug delivery device 1000 in response to confirmation of premedication. Some drugs deliverable from the drug delivery device 1000, such as various oncology drugs, can require that a user receive a premedication drug before the drug is delivered to the user from the drug delivery device 1000. The accessory 1006 can be configured to receive confirmation that the premedication drug has been administered to the user and, in response to receiving the confirmation, automatically allow the accessory 1006 to be released from drug delivery device 1000. Until the accessory 1006 receives confirmation that the premedication drug has been administered to the user, drug delivery from the drug delivery device 1000 remains locked. The premedication drug can be administered to a user in any of a variety of ways, such as by the user taking a pill, the user receiving an injection, etc.

The accessory 1006 can be configured to receive confirmation of premedication in a variety of ways. For example, the drug delivery device 1000, the accessory 1006, and/or a mobile phone or other external device that is separate from an external to each of the drug delivery device 1000 and the accessory 1006 can include a mechanical or electrical button, lever, switch, or other mechanism configured to be actuated, e.g., pressed, flipped, etc., by a user to indicate that the user has received a premedication. In some embodiments, the external device is a drug delivery device configured to deliver the premedication drug to the user. In other embodiments, the external device is a mobile phone or other computing device. The button, lever, switch, or other mechanism is in operative communication with the accessory's electronics, e.g., a processor thereof, such that the electronics is configured to receive input indicative of the actuation of the button, lever, switch, or other mechanism and thus receive confirmation of premedication. For another example, the drug delivery device 1000, the drug delivery device 1000, the accessory 1006, and/or a mobile phone or other external device that is separate from an external to each of the drug delivery device 1000 and the accessory 1006 can be configured to provide an audio query, e.g., via a speaker, etc., asking if premedication has been administered to the user. In some embodiments, the external device is a drug delivery device configured to deliver the premedication drug to the user. In other embodiments, the external device is a mobile phone or other computing device. The drug delivery device 1000, the accessory 1006, and/or the mobile phone or other external device can be configured to receive an audio response in reply to the audio query, e.g., a spoken "yes" or "no." The electronics of the accessory 1006 is configured to determine, based on the reply to the audio query, whether or not to unlock the drug delivery device 1000 for drug delivery. A "yes" reply allows for drug delivery since premedication has been administered to the user. A "no" reply does not allow for drug delivery since premedication has not been administered to the user. Instead of receiving an audio reply, a reply can be received in another way, such as via a button, lever, switch, or other mechanism similar to that discussed above. Instead of the query being provided as an audio query, the query can be provided in another way. For example, a query can be provided message on a display of the drug delivery device 1000, the accessory 1006, and/or the mobile phone or other external device. For another example, a query can be provided as a message written on the drug delivery device 1000 and/or the accessory 1006 either directly such as by etching, printing, etc. or indirectly such as on a sticker, label, etc. adhered to the drug delivery device 1000 and/or the accessory 1006.

In addition to receiving confirmation of premedication, the electronics can be configured to receive additional data related to the premedication. The additional data can be used to determine that premedication was correctly administered and/or can be stored as data relevant to future treatment analysis. If the premedication was not correctly administered, drug delivery from the drug delivery device 1000 can remain locked. The additional data can be input in a variety of ways, such as by user input similar to that discussed above regarding user input that the premedication drug was administered.

One or more types of additional data can be acquired. For example, the additional data can include a day/time of the premedication administration. If the premedication drug was administered within a predetermined threshold amount of time with the current date/time, then drug delivery from the drug delivery device 1000 is allowed. If the premedication drug was not administered within the predetermined threshold amount of time with the current date/time, then drug delivery from the drug delivery device 1000 is prevented. Checking timing of premedication drug administration may help ensure that the premedication drug has had sufficient time to take desired effect, e.g., to allow for sufficient absorption or dissolution to reach a sufficient blood concentration, before drug is delivered from the drug delivery device 1000. For another example, the additional data can include a brand of the premedication. If the brand is an acceptable brand, then drug delivery from the drug delivery device 1000 is allowed. If the brand is not an acceptable brand, then drug delivery from the drug delivery device 1000 is prevented. An acceptable brand is a preapproved brand stored in a memory. Checking acceptability of a brand may help ensure that the user received an approved formulation of the premedication and/or received the particular premedication drug prescribed to the user. For yet another example, the additional data can include an amount of the premedication drug that was administered. If the amount of premedication drug administered is within a predetermined threshold amount, then drug delivery from the drug delivery device 1000 is allowed. If the amount of premedication drug administered is not within the predetermined threshold amount, then drug delivery from the drug delivery device 1000 is prevented. Checking the amount of premedication drug administered may help ensure that enough of the premedication drug has been administered, e.g., that the user's prescribed amount of premedication drug has been administered, before drug is delivered from the drug delivery device 1000.

In embodiments in which the accessory 1006 does not directly receive confirmation of premedication delivery, such as by a user input being provided directly to the accessory 1006, the accessory 1006 can be configured to receive confirmation of premedication delivery directly from the drug delivery device 1000 or the mobile phone or other external device that directly receives the confirmation of premedication delivery. Alternatively, the drug delivery device 1000 or the mobile phone or other external device that directly receives the confirmation of premedication delivery can be configured to communicate confirmation of premedication delivery to another device that communicates the confirmation of premedication delivery to the accessory 1006. For example, the drug delivery device 1000 can receive the confirmation of premedication delivery and communicate confirmation of premedication delivery to the mobile phone or other external device, which then communicates confirmation of premedication delivery to the accessory 1006. For another example, the mobile phone or other external device can receive the confirmation of premedication delivery and communicate confirmation of premedication delivery to an external computer system, e.g., via an app installed on the mobile phone or other external device, which then communicates confirmation of premedication delivery to the accessory 1006.

For yet another example, the electronics of the accessory 1006 can be configured to cause the accessory 1006 to unlock from the drug delivery device 1000 in response to an instruction from an external computer system. In this way, the accessory 1006 and the drug delivery device 1000 can be configured to be remotely unlocked. The electronics of the accessory 1006 can include a communications interface configured to communicate with the external computer system, e.g., via a communications interface of the external computer system, and to receive the instruction.

The instruction can be transmitted from the external computer system to the accessory 1006 based on occurrence of a predetermined trigger event. The occurrence of the predetermined trigger event can automatically cause the instruction to be transmitted from the external computer system to the accessory 1006 or can serve as a cue to a person, e.g., a health care provider or other authorized user, to manually cause the instruction to be transmitted from the external computer system to the accessory 1006. Manually causing the instruction to be transmitted may allow for the predetermined trigger event to be verified by a human before the accessory 1006 and the drug delivery device 1000 are unlocked. The predetermined trigger event can be any one or more of a variety of events. For example, the predetermined trigger event can include a sensed geographic location of the drug delivery device 1000 and/or the accessory 1006 being an acceptable geographic location, similar to that discussed above regarding the accessory 1006 including a location sensor. For another example, the predetermined trigger event can include a sensed time being an acceptable time, similar to that discussed above regarding the accessory 1006 including a time sensor. For yet another example, the predetermined trigger event can include a sensed biometric input being an acceptable biometric input, similar to that discussed above regarding unlocking the accessory 1006 from the drug delivery device 1000 in response to a biometric input. In some embodiments, instead of an accessory including electronics configured to cause the accessory to unlock from a drug delivery in response to an instruction from an external computer system, the drug delivery device can include electronics configured to unlock the accessory and the drug delivery device similar to that discussed above regarding the accessory 1006 including the electronics.

The drug delivery device 400 of FIG. 4 is also an exemplary embodiment of the drug delivery device 1000 of FIG. 11.

In embodiments in which an accessory and/or a drug delivery device includes electronics, the electronics can include a communications interface configured to communicate data to an external computer system, e.g., via a communications interface of the external computer system, indicating that the drug delivery device and the accessory have been decoupled from one another. The electronics can also include a processor configured to determine that the decoupling has occurred, such as by a sensor (e.g., a motion sensor, a Hall effect sensor, etc.) communicating data to the processor indicative of an unlocked state. A user's health care provider can have access to the external computer system. The user's health care provider can thus be informed via the external computer system, e.g., via information shown on a display, information provided in an email, information provided in a text message, information entered into the user's electronic health record, etc., when the drug delivery device and the accessory have been decoupled from one another so as to indicate a presumption that the drug delivery device is being used for drug delivery. The health care provider may therefore be better able to analyze user treatment and/or user compliance and thereby improve user care.

As discussed herein, one or more aspects or features of the subject matter described herein, e.g., electronic components such as a processor, memory, communications interface, etc., can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computer system may include clients and servers. A client and server are generally remote from each other and typically interact through a communications network, e.g., the Internet, a wireless wide area network, a local area network, a wide area network, or a wired network. The relationship of client and server arises by virtue of computer programs running on the respective computers and having a client-server relationship to each other.

The computer programs, which can also be referred to as programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein, for example a user interface, can be implemented on a computer having a display screen, such as for example a cathode ray tube (CRT) or a liquid crystal display (LCD) or a light emitting diode (LED) monitor for displaying information to the user. The display screen can allow input thereto directly (e.g., as a touch screen) or indirectly (e.g., via an input device such as a keypad or voice recognition hardware and software). Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input.

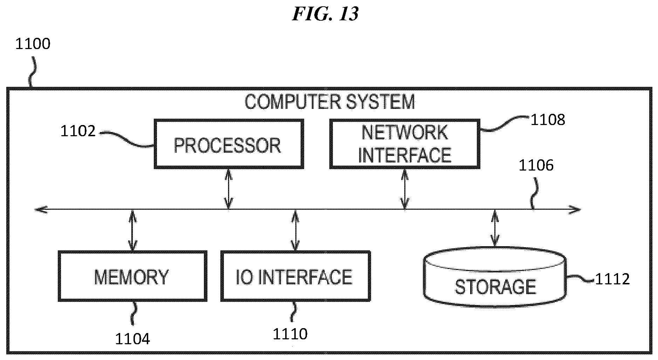

FIG. 13 illustrates one exemplary embodiment of a computer system 1100. The computer system 1100 includes a processor 1102 configured to control the operation of the computer system 1100. The processor 1102 can include any type of microprocessor or central processing unit (CPU), including programmable general-purpose or special-purpose microprocessors and/or any one of a variety of proprietary or commercially available single or multi-processor systems. The computer system 1100 also includes a memory 1104 configured to provide temporary storage for code to be executed by the processor 1102 or for data acquired from one or more users, storage devices, sensors, and/or databases. The memory 1104 can include read-only memory (ROM), flash memory, one or more varieties of random access memory (RAM) (e.g., static RAM (SRAM), dynamic RAM (DRAM), or synchronous DRAM (SDRAM)), and/or a combination of memory technologies.

The various elements of the computer system 1100 are coupled to a bus system 1106. The illustrated bus system 1106 is an abstraction that represents any one or more separate physical busses, communication lines/interfaces, and/or multi-drop or point-to-point connections, connected by appropriate bridges, adapters, and/or controllers. The computer system 1100 also includes a network interface 1108 (also referred to herein as a communications interface), an input/output (IO) interface(s) 1110, and a storage device 1112.

The communications interface 1108 is configured to enable the computer system to communicate with remote devices, e.g., other computer systems, and can be, for example, remote desktop connection interfaces, Ethernet adapters, and/or other local area network (LAN) adapters. The IO interface 1110 includes one or more interface components to connect the computer system 1100 with other electronic equipment. For example, the IO interface 1110 can include high speed data ports, such as universal serial bus (USB) ports, 1394 ports, Wi-Fi, Bluetooth, etc. Additionally, the computer system 1100 can be accessible to a human user, and thus the IO interface 1110 can include displays, speakers, keyboards, pointing devices, and/or various other video, audio, or alphanumeric interfaces. The storage device 1112 includes any conventional medium for storing data in a non-volatile and/or non-transient manner. The storage device 1112 is thus configured to hold data and/or instructions in a persistent state in which the value(s) are retained despite interruption of power to the computer system. The storage device 1112 can include one or more hard disk drives, flash drives, USB drives, optical drives, various media cards, diskettes, compact discs, and/or any combination thereof and can be directly connected to the computer system or remotely connected thereto, such as over a network. In an exemplary embodiment, the storage device 1112 includes a tangible or non-transitory computer readable medium configured to store data, e.g., a hard disk drive, a flash drive, a USB drive, an optical drive, a media card, a diskette, or a compact disc.

The elements illustrated in FIG. 13 can be some or all of the elements of a single physical machine. In addition, not all of the illustrated elements need to be located on or in the same physical machine.

The computer system 1100 can include a web browser for retrieving web pages or other markup language streams, presenting those pages and/or streams (visually, aurally, or otherwise), executing scripts, controls and other code on those pages/streams, accepting user input with respect to those pages/streams (e.g., for purposes of completing input fields), issuing HyperText Transfer Protocol (HTTP) requests with respect to those pages/streams or otherwise (e.g., for submitting to a server information from the completed input fields), and so forth. The web pages or other markup language can be in HyperText Markup Language (HTML) or other conventional forms, including embedded Extensible Markup Language (XML), scripts, controls, and so forth. The computer system 1100 can also include a web server for generating and/or delivering the web pages to client computer systems.

The computer system 1100 can also include any of a variety of other software and/or hardware components, including by way of example, operating systems and database management systems. Although an exemplary computer system is depicted and described herein, it will be appreciated that this is for sake of generality and convenience. In other embodiments, the computer system 1100 may differ in architecture and operation from that shown and described here. For example, the memory 1104 and storage device 1112 can be integrated together or the communications interface 1108 can be omitted if communication with another computer system is not necessary.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that the scope of the invention is defined by the claims.

## Claims

1. A drug delivery system, comprising:
a drug delivery device (100, 202) comprising
a dispensing head (110) including a tip configured to be positioned in a nose of a patient, the tip having an opening (108) therein,
a drug holder (102) containing a drug therein, and
an actuator (106) configured to be actuated by a user to cause the drug to be delivered out of the opening;
an accessory (204) comprising a locking mechanism (208) configured to releasably couple to the drug delivery device;
wherein, with the locking mechanism and the drug delivery device releasably coupled together, the drug delivery device and the accessory are a mated unit and the drug cannot exit through the opening into the nose of the patient; and
wherein, with the locking mechanism released from the drug delivery device, the accessory is a separate, independent member from the drug delivery device and the actuator is configured to be actuated to cause the drug to be delivered out of the opening into the nose of the patient; and
**characterised in that** at least one of the drug delivery device and the accessory includes a communications interface (120) configured to communicate with an external computer system; and
the communications interface is configured to receive an instruction from the external computer that allows the locking mechanism to be released from the drug delivery device.

2. A drug delivery system, comprising:
a drug delivery device (100, 202) comprising
a dispensing head (110) including a tip configured to be positioned in a nose of a patient, the tip having an opening (108) therein,
a drug holder (102) containing a drug therein, and
an actuator (106) configured to be actuated by a user to cause the drug to be delivered out of the opening;
an accessory (204) comprising a locking mechanism (208) configured to releasably couple to the drug delivery device;
wherein, with the locking mechanism and the drug delivery device releasably coupled together, the drug delivery device and the accessory are a mated unit and the drug cannot exit through the opening into the nose of the patient; and
wherein, with the locking mechanism released from the drug delivery device, the accessory is a separate, independent member from the drug delivery device and the actuator is configured to be actuated to cause the drug to be delivered out of the opening into the nose of the patient; and **characterised in that** either;
the accessory further comprises a time sensor, wherein the locking mechanism includes a processor configured to automatically release the locking mechanism from the drug delivery device in response to time data gathered by the time sensor indicating that the time data is a predetermined acceptable time for delivery of the drug from the drug delivery device; and the accessory further comprises a memory storing predetermined acceptable times therein, the memory being accessible to the processor; or
the accessory further comprises a location sensor, wherein the locking mechanism includes a processor configured to allow the locking mechanism to be released from the drug delivery device in response to geographic location data gathered by the location sensor indicating that the geographic location data is a predetermined acceptable geographic location for delivery of the drug from the drug delivery device; and the accessory further comprises a memory storing predetermined acceptable geographic locations therein, the memory being accessible to the processor.

3. The system of claim 1 or claim 2, wherein, with the locking mechanism and the drug delivery device releasably coupled together, the accessory covers the opening.

4. The system of any preceding claim, wherein the drug delivery device includes a depression (308) formed in the dispensing head; and
the locking mechanism includes a protrusion (310) extending from the accessory that is configured to be releasably seated in the depression, and optionally wherein the depression (308) is a device indicator configured to present information to a user about a status of drug delivery from the drug delivery device.

5. The system of claim 1 or claim 2, wherein the drug delivery device further comprising a depth guide (610);
the dispensing head includes a distal base (612); and
with the locking mechanism and the drug delivery device releasably coupled together, the accessory is located between the depth guide and the distal base.

6. The system of any preceding claim, wherein the accessory is a box that contains a partial portion of the drug delivery device therein when releasably coupled to the drug delivery device, or wherein the accessory is a box that fully contains the drug delivery device therein when releasably coupled to the drug delivery device.

7. The system of claim 1 or claim 2, wherein the accessory includes a cavity formed therein; and
with the locking mechanism and the drug delivery device releasably coupled together, the drug delivery device is seated in the cavity and extends proximally from the accessory.

8. The system of claim 7, wherein the actuator is configured to be seated in the cavity.

9. The system of claim 7, wherein the accessory includes a measurement mechanism configured to facilitate a determination of an amount of drug contained in the drug holder.

10. The system of claim 9, wherein the measurement mechanism includes a spring configured to be compressed with the drug delivery device seated in the cavity, an amount of the compression being indicative of the amount of the drug contained in the drug holder, or wherein the drug holder includes a magnet thereon; and
the measurement mechanism includes a Hall effect sensor.

11. The system of claim 1, wherein the instruction is based on at least one of the drug delivery device and the accessory being in an acceptable geographic location.

12. The system of claim 1, wherein the instruction is based on occurrence of a predetermined acceptable time for delivery of the drug from the drug delivery device.

13. The system of claim 1, wherein the instruction is based on an acceptable biometric input being input to at least one of the drug delivery device and the accessory.

14. The system of any preceding claim, wherein the releasable coupling is mechanical, or wherein the releasable coupling is electrical.

15. The system of any preceding claim, wherein the drug is one of ketamine, esketamine, naloxone, and sumatriptan.

## Patentansprüche

1. Arzneimittelabgabevorrichtung, umfassend:
Arzneimittelabgabevorrichtung (100, 202), umfassend
einen Abgabekopf (110), der eine Spitze einschließt, die konfiguriert ist, um in einer Nase eines Patienten positioniert zu werden, wobei die Spitze eine Öffnung (108) darin aufweist,
einen Arzneimittelhalter (102), der darin ein Arzneimittel enthält, und
einen Aktuator (106), der konfiguriert ist, um betätigt zu werden, um die Abgabe des Arzneimittels aus der Öffnung zu bewirken;
ein Zubehör (204), das einen Verriegelungsmechanismus (208) umfasst, der konfiguriert ist, um lösbar an die Arzneimittelabgabevorrichtung zu koppeln;
wobei, wenn der Verriegelungsmechanismus und die Arzneimittelabgabevorrichtung lösbar miteinander gekoppelt sind, die Arzneimittelabgabevorrichtung und das Zubehör eine gepaarte Einheit sind und das Arzneimittel nicht durch die Öffnung in die Nase des Patienten austreten kann; und
wobei, wenn der Verriegelungsmechanismus von der Arzneimittelabgabevorrichtung gelöst wird, das Zubehör ein separates, von der Arzneimittelabgabevorrichtung unabhängiges Element ist und der Aktuator konfiguriert ist, um betätigt zu werden, um zu bewirken, dass das Arzneimittel aus der Öffnung in die Nase des Patienten abgegeben wird; und
**dadurch gekennzeichnet, dass** mindestens eines von der Arzneimittelabgabevorrichtung und dem Zubehör eine Kommunikationsschnittstelle (120) einschließt, die konfiguriert ist, um mit einem externen Computersystem zu kommunizieren; und
die Kommunikationsschnittstelle konfiguriert ist, um eine Anweisung von dem externen Computer zu empfangen, die es ermöglicht, dass der Verriegelungsmechanismus von der Arzneimittelabgabevorrichtung gelöst wird.

2. Arzneimittelabgabevorrichtung, umfassend:
Arzneimittelabgabevorrichtung (100, 202), umfassend
einen Abgabekopf (110), der eine Spitze einschließt, die konfiguriert ist, um in einer Nase eines Patienten positioniert zu werden, wobei die Spitze eine Öffnung (108) darin aufweist,
einen Arzneimittelhalter (102), der darin ein Arzneimittel enthält, und
einen Aktuator (106), der konfiguriert ist, um betätigt zu werden, um die Abgabe des Arzneimittels aus der Öffnung zu bewirken;
ein Zubehör (204), das einen Verriegelungsmechanismus (208) umfasst, der konfiguriert ist, um lösbar an die Arzneimittelabgabevorrichtung zu koppeln;
wobei, wenn der Verriegelungsmechanismus und die Arzneimittelabgabevorrichtung lösbar miteinander gekoppelt sind, die Arzneimittelabgabevorrichtung und das Zubehör eine gepaarte Einheit sind und das Arzneimittel nicht durch die Öffnung in die Nase des Patienten austreten kann; und
wobei, wenn der Verriegelungsmechanismus von der Arzneimittelabgabevorrichtung gelöst wird, das Zubehör ein separates, von der Arzneimittelabgabevorrichtung unabhängiges Element ist und der Aktuator konfiguriert ist, um betätigt zu werden, um zu bewirken, dass das Arzneimittel aus der Öffnung in die Nase des Patienten abgegeben wird; und
**dadurch gekennzeichnet, dass** entweder;
das Zubehör ferner einen Zeitsensor umfasst, wobei der Verriegelungsmechanismus einen Prozessor einschließt, der konfiguriert ist, um den Verriegelungsmechanismus automatisch von der Arzneimittelabgabevorrichtung zu lösen als Reaktion auf Zeitdaten, die von dem Zeitsensor gesammelt werden und anzeigen, dass die Zeitdaten eine vorbestimmte akzeptable Zeit für die Abgabe des Arzneimittels von der Arzneimittelabgabevorrichtung sind; und das Zubehör ferner einen Speicher umfasst, der darin vorbestimmte akzeptable Zeiten speichert, wobei der Speicher für den Prozessor zugänglich ist; oder
das Zubehör ferner einen Positionssensor umfasst, wobei der Verriegelungsmechanismus einen Prozessor einschließt, der konfiguriert ist, um zu ermöglichen, dass der Verriegelungsmechanismus von der Arzneimittelabgabevorrichtung gelöst wird, als Reaktion darauf, dass von dem Positionssensor gesammelte geografische Positionsdaten anzeigen, dass die geografischen Positionsdaten eine vorbestimmte akzeptable geografische Position für die Abgabe des Arzneimittels von der Arzneimittelabgabevorrichtung sind; und das Zubehör ferner einen Speicher umfasst, der darin vorbestimmte akzeptable geografische Positionen speichert, wobei der Speicher für den Prozessor zugänglich ist.

3. System nach Anspruch 1 oder 2, wobei, wenn der Verriegelungsmechanismus und die Arzneimittelabgabevorrichtung lösbar miteinander gekoppelt sind, das Zubehör die Öffnung abdeckt.

4. System nach einem der vorstehenden Ansprüche, wobei die Arzneimittelabgabevorrichtung eine in dem Abgabekopf ausgebildete Vertiefung (308) einschließt; und
der Verriegelungsmechanismus einen Vorsprung (310) einschließt, der sich von dem Zubehör erstreckt und konfiguriert ist, um lösbar in der Vertiefung aufgenommen zu werden, und optional wobei die Vertiefung (308) ein Vorrichtungsindikator ist, der konfiguriert ist, um einem Benutzer Informationen über einen Status der Arzneimittelabgabe von der Arzneimittelabgabevorrichtung zu präsentieren.

5. System nach Anspruch 1 oder 2, wobei die Arzneimittelabgabevorrichtung ferner eine Tiefenführung (610) umfasst;
der Abgabekopf eine distale Basis (612) einschließt; und
wenn der Verriegelungsmechanismus und die Arzneimittelabgabevorrichtung lösbar miteinander gekoppelt sind, das Zubehör zwischen der Tiefenführung und der distalen Basis angeordnet ist.

6. System nach einem der vorstehenden Ansprüche, wobei das Zubehör eine Box ist, die einen Teilbereich der Arzneimittelabgabevorrichtung darin enthält, wenn sie lösbar an die Arzneimittelabgabevorrichtung gekoppelt ist, oder wobei das Zubehör eine Box ist, die die Arzneimittelabgabevorrichtung vollständig darin enthält, wenn sie lösbar an die Arzneimittelabgabevorrichtung gekoppelt ist.

7. System nach Anspruch 1 oder 2, wobei das Zubehör einen darin ausgebildeten Hohlraum einschließt;
und
wenn der Verriegelungsmechanismus und die Arzneimittelabgabevorrichtung lösbar miteinander gekoppelt sind, die Arzneimittelabgabevorrichtung in dem Hohlraum angeordnet sind und sich proximal von dem Zubehör erstreckt.

8. System nach Anspruch 7, wobei der Aktuator konfiguriert ist, um in dem Hohlraum angeordnet zu werden.

9. System nach Anspruch 7, wobei das Zubehör einen Messmechanismus einschließt, der konfiguriert ist, um eine Bestimmung einer Menge an Arzneimittel zu erleichtern, die in dem Arzneimittelhalter enthalten ist.

10. System nach Anspruch 9, wobei der Messmechanismus eine Feder einschließt, die konfiguriert ist, um mit der in dem Hohlraum angeordneten Arzneimittelabgabevorrichtung komprimiert zu werden, wobei ein Ausmaß der Kompression die Menge des in dem Arzneimittelhalter enthaltenen Arzneimittels anzeigt, oder wobei der Arzneimittelhalter einen Magneten darauf einschließt; und
der Messmechanismus einen Hall-Effekt-Sensor einschließt.

11. System nach Anspruch 1, wobei die Anweisung auf mindestens einer der Arzneimittelabgabevorrichtung und dem Zubehör basiert, die sich an einer akzeptablen geografischen Position befinden.

12. System nach Anspruch 1, wobei die Anweisung auf dem Auftreten einer vorbestimmten akzeptablen Zeit für die Abgabe des Arzneimittels aus der Arzneimittelabgabevorrichtung basiert.

13. System nach Anspruch 1, wobei die Anweisung auf einer akzeptablen biometrischen Eingabe basiert, die in mindestens eine der Arzneimittelabgabevorrichtung und des Zubehörs eingegeben wird.

14. System nach einem der vorstehenden Ansprüche, wobei die lösbare Kupplung mechanisch ist, oder wobei die lösbare Kupplung elektrisch ist.

15. System nach einem der vorstehenden Ansprüche, wobei das Arzneimittel eines von Ketamin, Esketamin, Naloxon oder Sumatriptan ist.

## Revendications

1. Système d'administration de médicament, comprenant :
un dispositif d'administration de médicament (100, 202) comprenant
une tête de distribution (110) comportant un embout conçu pour être positionné dans un nez d'un patient, l'embout ayant une ouverture (108) dans celui-ci,
un porte-médicament (102) contenant un médicament à l'intérieur de celui-ci, et
un actionneur (106) conçu pour être actionné par un utilisateur pour provoquer l'administration du médicament par l'ouverture ;
un accessoire (204) comprenant un mécanisme de verrouillage (208) conçu pour s'accoupler de manière libérable au dispositif d'administration de médicament ;
dans lequel, lorsque le mécanisme de verrouillage et le dispositif d'administration de médicament sont accouplés de manière amovible, le dispositif d'administration de médicament et l'accessoire forment une unité accouplée et le médicament ne peut pas sortir par l'ouverture dans le nez du patient ; et
dans lequel, lorsque le mécanisme de verrouillage est libéré du dispositif d'administration de médicament, l'accessoire est un élément séparé et indépendant du dispositif d'administration de médicament et l'actionneur est conçu pour être actionné pour provoquer l'administration du médicament par l'ouverture dans le nez du patient ; et
**caractérisé en ce qu'**au moins l'un parmi le dispositif d'administration de médicament et l'accessoire comporte une interface de communications (120) configurée pour communiquer avec un système informatique externe ; et
l'interface de communications est configurée pour recevoir une instruction à partir de l'ordinateur externe qui permet de libérer le mécanisme de verrouillage du dispositif d'administration de médicament.

2. Système d'administration de médicament, comprenant :
un dispositif d'administration de médicament (100, 202) comprenant
une tête de distribution (110) comportant un embout conçu pour être positionné dans un nez d'un patient, l'embout ayant une ouverture (108) dans celui-ci,
un porte-médicament (102) contenant un médicament à l'intérieur de celui-ci, et
un actionneur (106) conçu pour être actionné par un utilisateur pour provoquer l'administration du médicament par l'ouverture ;
un accessoire (204) comprenant un mécanisme de verrouillage (208) conçu pour s'accoupler de manière libérable au dispositif d'administration de médicament ;
dans lequel, lorsque le mécanisme de verrouillage et le dispositif d'administration de médicament sont accouplés de manière amovible, le dispositif d'administration de médicament et l'accessoire forment une unité accouplée et le médicament ne peut pas sortir par l'ouverture dans le nez du patient ; et
dans lequel, lorsque le mécanisme de verrouillage est libéré du dispositif d'administration de médicament, l'accessoire est un élément séparé et indépendant du dispositif d'administration de médicament et l'actionneur est conçu pour être actionné pour provoquer l'administration du médicament par l'ouverture dans le nez du patient ; et **caractérisé en ce que** soit ;
l'accessoire comprend en outre un capteur de temps, dans lequel le mécanisme de verrouillage comporte un processeur configuré pour libérer automatiquement le mécanisme de verrouillage à partir du dispositif d'administration de médicament en réponse aux données temporelles recueillies par le capteur de temps indiquant que les données temporelles correspondent à un temps acceptable prédéterminé pour l'administration du médicament à partir du dispositif d'administration de médicament ; et l'accessoire comprend en outre une mémoire sur laquelle sont stockées des temps acceptables prédéterminés, la mémoire étant accessible au processeur ; soit
l'accessoire comprend en outre un capteur de localisation, dans lequel le mécanisme de verrouillage comporte un processeur configuré pour permettre au mécanisme de verrouillage d'être libéré du dispositif d'administration de médicament en réponse aux données de localisation géographique recueillies par le capteur de localisation indiquant que les données de localisation géographique sont un emplacement géographique acceptable prédéterminé pour l'administration du médicament à partir du dispositif d'administration de médicament ; et l'accessoire comprend en outre une mémoire dans laquelle sont stockés des localisations géographiques acceptables prédéterminées, la mémoire étant accessible au processeur.

3. Système selon la revendication 1 ou la revendication 2, dans lequel, lorsque le mécanisme de verrouillage et le dispositif d'administration de médicament sont accouplés de manière amovible, l'accessoire couvre l'ouverture.

4. Système selon l'une quelconque revendication précédente, dans lequel le dispositif d'administration de médicament comporte une dépression (308) formée dans la tête de distribution ; et
le mécanisme de verrouillage comporte une saillie (310) qui s'étend à partir de l'accessoire qui est conçue pour se loger de manière amovible dans la dépression, et éventuellement dans lequel la dépression (308) est un indicateur de dispositif configuré pour présenter à un utilisateur des informations concernant un état de l'administration de médicament à partir du dispositif d'administration de médicament.

5. Système selon la revendication 1 ou la revendication 2, dans lequel le dispositif d'administration de médicament comprenant en outre un guide de profondeur (610) ;
la tête de distribution comporte une base distale (612) ; et
lorsque le mécanisme de verrouillage et le dispositif d'administration de médicament sont accouplés de manière amovible, l'accessoire est situé entre le guide de profondeur et la base distale.

6. Système selon l'une quelconque revendication précédente, dans lequel l'accessoire est une boîte qui contient une partie partielle du dispositif d'administration de médicament lorsqu'il est accouplé de manière amovible au dispositif d'administration de médicament, ou dans lequel l'accessoire est une boîte qui contient entièrement le dispositif d'administration de médicament lorsqu'il est accouplé de manière amovible au dispositif d'administration de médicament.

7. Système selon la revendication 1 ou la revendication 2, dans lequel l'accessoire comporte une cavité formée dans celui-ci ;
et
lorsque le mécanisme de verrouillage et le dispositif d'administration de médicament sont accouplés de manière amovible, le dispositif d'administration de médicament est logé dans la cavité et s'étend de manière proximale à partir de l'accessoire.

8. Système selon la revendication 7, dans lequel l'actionneur est conçu pour être logé dans la cavité.

9. Système selon la revendication 7, dans lequel l'accessoire comporte un mécanisme de mesure conçu pour faciliter une détermination d'une quantité de médicament contenue dans le porte-médicament.

10. Système selon la revendication 9, dans lequel le mécanisme de mesure comporte un ressort conçu pour être comprimé lorsque le dispositif d'administration de médicament est logé dans la cavité, une quantité de la compression indiquant la quantité du médicament contenue dans le porte-médicament, ou dans lequel le porte-médicament comporte un aimant sur celui-ci ; et
le mécanisme de mesure comporte un capteur à effet Hall.

11. Système selon la revendication 1, dans lequel l'instruction est basée sur le fait qu'au moins l'un parmi le dispositif d'administration de médicament et l'accessoire se trouvent dans une localisation géographique acceptable.

12. Système selon la revendication 1, dans lequel l'instruction est basée sur une occurrence d'un temps acceptable prédéterminé pour l'administration du médicament à partir du dispositif d'administration.

13. Système selon la revendication 1, dans lequel l'instruction est basée sur une entrée biométrique acceptable entrée pour au moins l'un parmi le dispositif d'administration de médicament et l'accessoire.

14. Système selon l'une quelconque revendication précédente, dans lequel l'accouplement libérable est mécanique, ou dans lequel l'accouplement libérable est électrique.

15. Système selon l'une quelconque des revendications précédentes, dans lequel le médicament est l'un parmi kétamine, eskétamine, naloxone et sumatriptan.
